# EUROPÄISCHE PATENTSCHRIFT

(11) **EP 2 748 148 B1**
(45) Veröffentlichungstag und Bekanntmachung des Hinweises auf die Patenterteilung: **08.06.2016**
(21) Anmeldenummer: 10812845.5
(22) Anmeldetag: 22.12.2010
(51) Int. Cl.: C07D 231/12

(54) **VERFAHREN ZUR REINIGUNG VON PYRAZOLEN**
METHOD FOR PURIFYING PYRAZOLES
PROCÉDÉ DE PURIFICATION DE PYRAZOLES

(30) Priorität: 23.12.2009 DE 102009060150
(43) Veröffentlichungstag der Anmeldung: 02.07.2014
(73) Patentinhaber: K+S Aktiengesellschaft, 34131 Kassel (DE)
(72) Erfinder: WALDMANN, Ludger, 48291 Telgte (DE); HAHN, Caspar-Friedrich, 48291 Telgte (DE); ECKENRATH, Helmut, 06766 Wolfen (DE); PASCH, Roland, 06749 Bitterfeld (DE)
(74) Vertreter: Reitstötter Kinzebach
(86) Internationale Anmeldenummer: PCT/DE2010/001514
(87) Internationale Veröffentlichungsnummer: WO 2011/076194

(56) Entgegenhaltungen:
- EP-A1- 0 974 585
- DE-A1- 4 328 228
- DE-A1- 19 631 764
- NOYCE ET AL.: "THE ULTRAVIOLET ABSORPTION SPECTRA OF SUBSTITUTED PYRAZOLES", J. ORG. CHEM., Bd. 20, Nr. 12, 1955, Seiten 1681-1686, XP002630674, DOI: 10.1021/jo01364a013
- VON AUWERS, KOHLHAAS: "I. Zur Spektrochemie heterocyclischer stickstoffhaltiger Verbindungen", JUSTUS LIEBIGS ANNALEN DER CHEMIE, Bd. 437, Nr. 1, 1924, Seiten 36-63, XP002630650,
- Becker et al.: "Organikum / Organisch-Chemisches Grundpraktikum", 1981, VEB Deutscher Verlag der Wissenschaften, Berlin, XP002630680, Seiten 743-744, Paragraph 2.4.5: Darstellung der Pikrate. Zitiert als allgemeines Fachwissen im Hinblick auf die zuvorgenannten Dokumente D1, D2.
- BUCHNER, FRITSCH: "VI. Darstellung und Derivate des freien Pyrazols", JUSTUS LIEBIGS ANNALEN DER CHEMIE, Bd. 273, 1893, Seiten 256-266, XP002630610,
- Kirschke: "1H-Pyrazole" In: Schaumann et al. (Hrsg.): "Methoden der organischen Chemie (Houben-Weyl), Band E8b "Hetarene III / Teil 2", Seiten 399-401 und 408-421", 29. November 1994 (1994-11-29), Georg Thieme Verlag, Stuttgart, XP002630611, Das ganze Dokument. Zitiert als allgemeines Fachwissen.

## Beschreibung

Die Erfindung betrifft ein Verfahren zur Reinigung von Pyrazolen und die Herstellung von Säureadditionssalzen von Pyrazolen, die weitestgehend frei von Nebenprodukten sind.

Die Verwendung von Pyrazolverbindungen wie 3,4-Dimethylpyrazol (DMP) und deren Säureadditionssalzen als Nitrifikationsinhibitoren zur Behandlung von Mineraldüngemitteln ist bekannt. So beschreibt die EP-A-1 120 388 die Herstellung und den Einsatz von 3,4-Dimethylpyrazoliumdihydrogenphosphat (DMPP) als Nitrifikationsinhibitor zur Behandlung von Mineraldüngemitteln. Die Verbindung Dimethylpyrazol selbst ist seit über 100 Jahren bekannt.

Die zweistufige Herstellung von Pyrazolen ausgehend von Carbonylverbindungen durch Umsetzung mit Ameisesäureestern und Umsetzung des gebildeten Zwischenproduktes mit Hydrazinen ist an sich bekannt.

Bei der Synthese von 3,4-Dimethylpyrazol durch Umsetzung von 2-Butanon und Methylformiat und nachfolgende Umsetzung des Zwischenproduktes mit Hydrazin wird neben 3,4-Dimethylpyrazol auch 3-Ethylpyrazol als Nebenprodukt gebildet. Dies ist darauf zurückzuführen, dass das durch Reaktion von 2-Butanon und Alkylformiat erhaltene 2-Methyl-3-oxobutanal durch 3-Oxopentanal verunreinigt ist. Die Reindarstellung von 2-Methyl-3-oxobutanal ist in der Literatur beschrieben, siehe beispielsweise Ann. d. Chem. 1924, 437, 43. Das Problem der Verunreinigung von DMP durch 3-Ethylpyrazol ist durch Destillation des Eduktes 2-Methyl-3-oxobutanol oder des Produktes DMP lösbar. Diese Verfahren sind jedoch aufwendig und führen zu stark erniedrigten Ausbeuten.

Eine weitere Möglichkeit der Abtrennung von 3-Ethylpyrazol ist eine Aufreinigung des 2-Methyl-3-oxobutanals durch Abfiltrieren des bei der Synthese anfallenden Natriumsalzes. Der Ketoaldehyd wird anschließend durch Ansäuern des Salzes in situ freigesetzt. Der Gehalt an 3-Ethypyrazol kann so deutlich gesenkt werden, wobei jedoch auch die Gesamtausbeute an DMP stark abfällt.

Die EP-A-0 974 585 beschreibt ein entsprechendes Verfahren zur Herstellung von Pyrazolen. Dabei wird das nach der ersten Umsetzung erhaltene Zwischenprodukt abfiltriert oder abzentrifugiert, wodurch sich der Gehalt von beispielsweise 3-Ethylpyrazol im letztendlich erhaltenen 3,4-Dimethylpyrazol signifikant vermindern kann. In einem Vergleichsbeispiel ist die Gesamtumsetzung ohne Aufreinigung oder Abtrennung des Zwischenproduktes beschrieben.

Aufgabe der vorliegenden Erfindung ist die Bereitstellung eines verbesserten Verfahrens zur Reinigung von Pyrazolen, insbesondere zur Abtrennung von 3-Ethylpryrazol von 3,4-Dimethylpyrazol und eines Verfahrens zur Herstellung von Pyrazolen durch Umsetzung von Carbonylverbindungen mit Ameisensäureestern und nachfolgend Hydrazinen, wobei die Nachteile der bekannten Verfahren vermieden werden sollen. Die gewünschten Pyrazole - werden damit ohne großen Aufwand in hohen Ausbeuten und weitestgehend frei von Nebenprodukten zugänglich.

Die Aufgabe wird erfindungsgemäß gelöst durch ein Verfahren zur Reinigung von Pyrazolen der allgemeinen Formel (I) 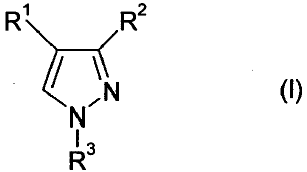 mit der Bedeutung
R¹, R² unabhängig voneinander Wasserstoff, Halogen, Nitro oder gegebenenfalls durch C₁₋₄-Alkyl, Halogen und/oder Nitro substituiertes C₁₋₂₀-Alkyl, C₃₋₈-Cycloalkyl, C₆₋₁₂-Aryl, C₇₋₁₃-Alkylaryl oder C₇₋₁₃-Aralkyl,
R³ Wasserstoff oder gegebenenfalls durch C₁₋₄-Alkyl, Halogen und/oder Nitro substituiertes C₁₋₂₀-Alkyl, C₃₋₈-Cycloalkyl, C₆₋₁₂-Aryl, C₇₋₁₃-Alkylaryl oder C₇₋₁₃-Aralkyl, durch

(1) Umsetzung von Carbonylverbindungen der allgemeinen Formel R¹-CH₂-CO-R² mit den angegebenen Bedeutungen für R¹ und R² in Gegenwart einer starken Base mit Ameisensäureestern der allgemeinen Formel H-COOR⁴, wobei R⁴ C₁₋₈-Alkyl bedeutet, oder mit Kohlenmonoxid,
(2) nachfolgende Umsetzung des gebildeten Zwischenproduktes mit Hydrazinen der allgemeinen Formel R³-NH-NH₂ mit der angegebenen Bedeutung für R³ in Gegenwart anorganischer oder organischer Säuren, und
(3) Lösen der so erhaltenen Pyrazole der allgemeinen Formel (I) in Wasser oder vorzugsweise einem organischen Lösungsmittel, nachfolgende Umsetzung mit mindestens äquimolaren Mengen ortho-Phosphorsäure oder Oxalsäure und Abtrennen der erhaltenen Säureadditionssalze durch Kristallisation;
wobei das Zwischenprodukt aus Schritt (1) ohne Abtrennung vom Reaktionsgemisch oder weitere Aufreinigung direkt im Schritt (2) weiter umgesetzt wird.

Es wurde erfindungsgemäß gefunden, dass die Aufreinigung von Pyrazolen in unaufwendiger Weise dadurch gelingt, dass die Pyrazole in Säureadditionssalze überführt werden, die aus organischen Lösungsmitteln ausgefällt bzw. kristallisiert werden. Die nicht gewünschten Nebenprodukte fallen bei der gezielten Kristallisation nicht mit aus, so dass beispielsweise die Abtrennung von 3-Ethylpyrazol-Säureadditionssalz von 3,4-Dimethylpyrazol-Säureadditionssalz durch gezielte Kristallisation gelingt. Damit ist eine Aufreinigung oder Abtrennung des Zwischenproduktes aus Schritt (1), wie sie in EP-A-0 974 585 beschrieben ist, nicht mehr notwendig.

Erfindungsgemäß wird das Zwischenprodukt aus Schritt (1) ohne Abtrennung vom Reaktionsgemisch oder weitere Aufreinigung direkt in Schritt (2) weiter umgesetzt.

Das Produkt aus Schritt (2) kann gegebenenfalls ohne Abtrennung vom Reaktionsgemisch oder weitere Aufreinigung direkt im Schritt (3) weiter umgesetzt werden.

Alternativ ist es erfindungsgemäß möglich, das in Schritt (2) erhaltene Pyrazol weiter aufzureinigen oder vom organischen Lösungsmittel abzutrennen, bevor es in Schritt (3) gereinigt wird. Sofern in Schritt (3) ein anderes Lösungsmittel als in Schritt (2) verwendet wird, ist es bevorzugt, das organische Lösungsmittel aus Schritt (2) vor der Aufreinigung in Schritt (3) abzutrennen.

Vorzugsweise werden aus dem Produkt aus Schritt (2) jedoch wasserlösliche Salze der anorganischen oder organischen Säuren abgetrennt, und das resultierende Produkt wird in Schritt(3) weiter umgesetzt.

Bei der Umsetzung in Schritt (2) entstehen wasserlösliche Salze der anorganischen oder organischen Säuren, die in Schritt (2) eingesetzt werden. Mit den starken Basen, die in Schritt (1) eingesetzt werden, bilden sich Salze. Beispielsweise bildet sich beim Zusatz von Schwefelsäure in Schritt (2) und Einsatz von Natriummethylat als Base in Schritt (1) Natriumsulfat, das vom Produkt aus Schritt (2) abgetrennt werden sollte, um zu Produkten mit hinreichender Reinheit zu gelangen.

Beim Einsatz der Alkali- oder Erdalkalimetallalkoholate in Schritt (1) werden diese häufig in Form alkoholischer Lösungen eingesetzt.

Das alkoholische Lösungsmittel wird nach Schritt (2) vorzugsweise durch Einengen des Reaktionsgemisches abgetrennt. Bevorzugt wird der erhaltene Rückstand sodann mit Wasser aufgenommen bzw. mit einer wässrigen Phase kontaktiert, um die wasserlöslichen Salze der anorganischen oder organischen Säuren in die wässrige Phase zu überführen. Häufig wird hierzu das Produktgemisch aus Schritt (2) mit einer Wasser-phase kontaktiert und mit einem zugesetzten organischen Lösungsmittel wie Petrolether oder Diethylether extrahiert.

Das organische Extraktionsmittel wird abgetrennt, nachdem zuvor gegebenenfalls über Trocknungsmittel, wie Natriumsulfat, getrocknet wurde. Die erhaltene organische Phase des Reaktionsproduktes wird dann in die Aufreinigung in Schritt (3) überführt.

Stören im Produkt des Schrittes (3) die Salze der organischen oder anorganischen Säure, beispielsweise Natriumsulfat, nicht, so kann auf die Aufarbeitung des Produktes aus Schritt (2) verzichtet werden. Durch das erfindungsgemäße Kristallisationsverfahren in Schritt (3) wird dennoch der Gehalt von beispielsweise 3-Ethylpyrazol in 3,4-Dimethylpyrazol signifikant vermindert.

Für eine insgesamt vorteilhafte Reinheit beispielsweise des 3,4-Dimethylpyrazols ist es jedoch vorteilhaft, dass aus Schritt (2) erhaltene Reaktionsgemisch einzuengen, den Rückstand mit Wasser aufzunehmen, die organische Phase abzutrennen und diese der Kristallisation zuzuführen.

Das erfindungsgemäße Verfahren zur Reinigung von Pyrazolen kann auf Pyrazole angewendet werden, die nach beliebigen Verfahren erhalten werden können. Vorzugsweise werden die Pyrazole jedoch durch das zweistufige Verfahren mit den vorstehend beschriebenen Schritten (1) und (2) erhalten. Die Umsetzungsbedingungen können dabei beliebig gewählt werden. Es ist beispielsweise möglich, die in EP-A-0 974 585 beschriebenen Bedingungen einzuhalten, wobei bevorzugt auf eine Abtrennung des Zwischenproduktes durch beispielsweise Abfiltrieren oder Abzentrifugieren verzichtet wird.

Als Ameisensäureester der allgemeinen Formel H-COOR⁴, wobei R⁴ C₁₋₈-Alkyl bedeutet, werden vorzugsweise C₁₋₃-Alkylformiate, insbesondere Methylformiat oder Ethylformiat, speziell Methylformiat, eingesetzt. Als Hydrazin der allgemeinen Formel R³-NH-NH₂ wird vorzugsweise Hydrazin eingesetzt. Auch Hydrazinsalze können eingesetzt werden.

Die Umsetzung in Schritt (1) erfolgt vorzugsweise bei einer Temperatur im Bereich von -20 bis 70 °C, besonders bevorzugt 0 bis 60 °C, insbesondere 20 bis 50 °C. Der Druck liegt vorzugsweise im Bereich von 1 bis 50 bar, besonders bevorzugt 1 bis 20 bar. Besonders bevorzugt erfolgt die Umsetzung bei Atmosphärendruck. Es kann auch vorteilhaft sein, bei unterhalb 35 °C, bzw. maximal 30 °C zu arbeiten. Eine Umsetzung mit Kohlenmonoxid als Carbonylquelle kann, wie in DE-A-198 31 656 beschrieben, erfolgen.

In dem zweiten Schritt beträgt die Temperatur bei der Umsetzung vorzugsweise 0 bis 90 °C, besonders bevorzugt 0 bis 70 °C, insbesondere 0 bis 40 °C. Der Druck beträgt vorzugsweise 1 bis 10 bar, besonders bevorzugt 1 bis 5 bar. Insbesondere wird bei Atmosphärendruck gearbeitet.

Die Molverhältnisse von Carbonylverbindungen zum Ameisensäureester und von Carbonylverbindung zur starken Base können in weiten Bereichen frei gewählt werden. Bevorzugt beträgt das Molverhältnis von Carbonylverbindung zum Ameisensäureester 0,1 bis 2 : 1, besonders bevorzugt 0,2 bis 1,5 : 1, insbesondere 0,3 bis 1,1 : 1. Das Molverhältnis von Carbonylverbindung zu starker Base beträgt vorzugsweise 0,1 bis 2 : 1, besonders bevorzugt 0,2 bis 1,5 : 1, insbesondere 0,3 bis 1,1 : 1.

Als starke Basen eignen sich beispielsweise Alkali- und Erdalkalimetallalkoholate. Bevorzugt werden Natrium- oder Kaliumalkoholate von C₁₋₄-Alkanolen eingesetzt. Besonders bevorzugt werden Natriummethylat oder Kaliummethylat eingesetzt.

Als Lösungsmittel für die Umsetzung kommen beispielsweise Alkohole, insbesondere C₁₋₄-Alkohole oder Glykole wie Ethylenglykol, Ether wie Diethylether, Tetrahydrofuran oder Dioxan in Betracht. Besonders bevorzugt erfolgt die Umsetzung in C₁₋₃-Alkoholen wie Methanol oder Ethanol.

In Schritt (2) erfolgt die Umsetzung mit Hydrazinen, die in der Regel als Hydrat eingesetzt werden, in Gegenwart einer anorganischen oder organischen Säure. Als Säure eignen sich anorganische oder organische Säuren, beispielsweise Schwefelsäure, Phosphorsäure, Sulfonsäuren, Ameisensäure, Essigsäure, Propionsäure, Oxalsäure oder Salzsäure. Besonders bevorzugt wird Schwefelsäure eingesetzt. Alternativ können auch die entsprechenden Salze der Hydrazine eingesetzt werden. Vorzugsweise wird der pH-Wert im Bereich von 6 bis 9, besonders bevorzugt 7 bis 8 gehalten.

Die Umsetzung in Schritt (2) kann ebenfalls in einem Lösungsmittel durchgeführt werden, das vorzugsweise dem Lösungsmittel des Schrittes (1) entspricht.

Das Molverhältnis des Zwischenproduktes des Schrittes (1) zu Hydrazin beträgt vorzugsweise 0,25 bis 2 : 1, besonders bevorzugt 0,5 bis 1,5 : 1, insbesondere 0,7 bis 1,25 : 1. Das Molverhältnis des Zwischenproduktes zur Säure beträgt vorzugsweise 0,1 bis 3,5 : 1, besonders bevorzugt 0,3 bis 3 : 1, insbesondere 0,5 bis 2,5 : 1.

Weitere Ausführungsformen des Verfahrens können der EP-A-0 974 585 entnommen werden, insbesondere den Absätzen [0013] bis [0018].

In einem bevorzugten Verfahren wird die Formylierungsreaktion in Schrittes (1) mit Methylformiat als Formylierungsreagenz bei einer Innentemperatur von maximal 30 °C durchgeführt. Bei der Folgereaktion in Schritt (2) wird Hydrazin-Hydrat so zudosiert, dass die Innentemperatur unter 10 °C liegt. Sodann wird etwa 50 gew.-%ige Schwefelsäure so zugegeben, dass die Innentemperatur unter 20 °C liegt. Es wird auf einen pH-Wert von etwa 7 eingestellt.

Eine stöchiometrische Reaktionsführung in Schritt (1) führt zu einem verminderten Verbrauch an Hydrazin in Schritt (2).

Gemäß einer Ausführungsform der Erfindung wird in Schritt (3) mit ortho-Phosphorsäure angesäuert, die vorzugsweise eine Konzentration von etwa 85 Gew.-% hat. Hierdurch kann es unmittelbar zur Bildung der entsprechenden phosphorsauren Pyrazoliumsalze (z.B. Pyrazoliumdihydrogenphosphat) kommen.

R¹, R² und R³ können unabhängig voneinander die vorstehend angegebenen Bedeutungen haben. Bevorzugt bedeuten R¹, R² und R³ unabhängig voneinander Wasserstoff oder C₁₋₃-Alkyl. Dabei bedeutet R³ vorzugsweise Wasserstoff.

Besonders bevorzugt bedeuten R¹ und R² unabhängig voneinander Wasserstoff, Methyl oder Ethyl, insbesondere Wasserstoff oder Methyl.

Besonders bevorzugt handelt es sich bei den Pyrazolen der allgemeinen Formel (I) um 3,4-Dimethylpyrazol, 3-Methylpyrazol, 4-Methylpyrazol, 3-Chlor-4-Methylpyrazol oder 4-Chlor-3-Methylpyrazol. Besonders bevorzugt ist das Pyrazol 3,4-Dimethylpyrazol.

In Schritt (3) kann auch Oxalsäure eingesetzt werden.

Das organische Lösungsmittel in Schritt (3) kann aus beliebigen geeigneten organischen Lösungsmitteln ausgewählt werden, die eine Ausfällung oder Kristallisation der

Säureadditionssalze der Pyrazole der allgemeinen Formel (I) erlauben. Besonders bevorzugt sind die organischen Lösungsmittel in Schritt (3) ausgewählt aus C₁₋₁₀-Alkanolen, aliphatischen C₃₋₁₀-Ketonen oder Gemischen davon. Besonders bevorzugt werden C₂₋₄-Alkanole, aliphatische C₃₋₅-Ketone oder Gemische davon, gegebenenfalls unter Zusatz von Wasser, eingesetzt. Besonders bevorzugt ist der Einsatz von Aceton, Ethanol oder Iso-Propanol oder Gemischen davon. Auch Wasser, wässrige Salzlösungen oder Gemische aus Wasser und organischem Lösungsmittel können eingesetzt werden. Bevorzugt wird ein organisches Lösungsmittel eingesetzt.

Die Ausfällung oder Kristallisation der Säure-Additionssalze in Schritt (3) kann bei beliebigen geeigneten Temperaturen durchgeführt werden. Um die Kristallisation zu fördern, ist es möglich die Temperatur in der Pyrazollösung abzusenken. Um eine Lösung der Pyrazole der allgemeinen Formel (I) im organischen Lösungsmittel zu erreichen, kann zuvor gegebenenfalls die Temperatur des Lösungsmittels erhöht werden.

Die Kristallisation oder Ausfällung kann in beliebiger geeigneter Weise durchgeführt werden. Es können auch mehrere Kristallisationsstufen neben- oder hintereinander geschaltet werden (siehe Beispiel 2). Beispielsweise kann eine fraktionierte Kristallisation durchgeführt werden. Entsprechende Verfahren sind dem Fachmann bekannt.

Das erfindungsgemäße Verfahren erlaubt es, die gewünschten Pyrazole der allgemeinen Formel (I) in sehr reiner Form als Säure-Additionssalze (Pyrazolium-Additionssalze) zu erhalten, wobei der Anteil an Nebenprodukten sehr stark verringert werden kann. Bei der Herstellung von 3,4-Dimethylpyrazol kann beispielsweise das als Nebenprodukt gebildete 3-Ethylpyrazol weitestgehend in Schritt (3) abgetrennt werden. Das Isomerenverhältnis von 3,4-Dimethylpyrazol zu 3-Ethylpyrazol beträgt vorzugsweise mehr als 95 : 5, besonders bevorzugt mehr als 97 : 3, insbesondere mehr als 98 : 2.

Die Erfindung wird durch die nachstehenden Beispiele näher erläutert.

### Beispiel 1:

### Synthese von 3,4-Dimethylpyrazol

**Edukte**

| Nr. | Name | Menge | Gehalt | MG | Mol | Mol % |
|---|---|---|---|---|---|---|
| 1 | Natriummethylat (30 %ig in Methanol) | 234 g | 30% | 54.02 | 1.30 | 140 |
| 2 | 2-Butanon | 72 g | | 72.11 | 1.00 | 100 |
| 3 | Methylformiat | 84 g | | 60.05 | 1.40 | 140 |
| 4 | Hydrazin-Hydrat | 50.06 g | | 50.05 | 1.00 | 100 |

### Durchführung:

In einem 2 L-Dreihalskolben mit KPG-Rührer, Anschütz, Innenthermometer, 250 mL-Tropftrichter und Rückflusskühler mit Hahnolive und Blasenzähler wurden unter einer Stickstoffatmosphäre 180 g 1 vorgelegt und innerhalb einer Stunde ein Gemisch aus 72 g 2 und 66 g 3 über den Tropftrichter so zugetropft, dass die Innentemperatur 30 °C nicht überstieg. Es wurde noch zwei Stunden bei 30 °C Badtemperatur nachgerührt. Anschließend wurden bei dieser Temperatur weitere 54 g 1 innerhalb von 15 Minuten zugetropft und danach weitere 18 g 3 innerhalb von 10 Minuten. Das Reaktionsgemisch wurde über Nacht bei Raumtemperatur gerührt. Die erhaltene Suspension wurde in einem Eisbad abgekühlt und bei T < 10 °C mit 65 mL 50 %iger Schwefelsäure auf pH = 7 eingestellt, wobei der Feststoff zum Teil in Lösung ging. Danach wurde im Eisbad bei T < 20 °C 50 g 4 zugetropft (stark exotherme Reaktion) und durch portionsweise Zugabe von 50 %iger Schwefelsäure (Verbrauch 20 mL) der pH-Wert zwischen 7 und 8 gehalten. Nach beendeter Zugabe wurde das Eisbad entfernt und über Nacht bei Raumtemperatur nachgerührt. Das Reaktionsgemisch wurde in einen 2 L-Rundkolben umgefüllt und der Reaktionskolben zweimal mit jeweils 50 mL destilliertem Wasser nachgewaschen. Mit 25 %iger Natriumhydroxid-Lösung wurde die Lösung auf pH = 8 eingestellt und das Methanol bei 30 °C Badtemperatur am Rotationsverdampfer entfernt.

Der Rückstand (ca. 300 mL) wurde mit 250 mL destilliertem Wasser und 500 mL Diethylether versetzt, in einen 2 L-Scheidetrichter überführt. Die organische Phase wurde abgetrennt und die wässrige Phase noch zweimal mit jeweils 50 mL Diethylether extrahiert. Die vereinigten organischen Phasen wurden einmal mit 150 mL destilliertem Wasser gewaschen, über Natriumsulfat getrocknet und am Rotationsverdampfer bei 30 °C Badtemperatur bis zur Gewichtskonstanz getrocknet. Ausbeute: 72,93 g gelbes Öl, das bei Raumtemperatur langsam kristallisiert.

Dieser Ansatz wurde noch einmal wiederholt und lieferte eine Ausbeute von 70,99 g. Mit Hilfe der 1 H-NMR-Spektroskopie wurde das Verhältnis von 3,4-Dimethylpyrazol zu 3-Ethylpyrazol in beiden Ansätzen zu 92 : 8 bestimmt. Neben 3,4-Dimethylpyrazol und 3-Ethylpyrazol ist auch eine größere Zahl nicht näher charakterisierter Verbindungen in geringeren Mengen im 1H-NMR-Spektrum sichtbar.

### Reinigung als Säure-Additionssalz

### Durchführung:

5,00 g rohes 3,4-Dimethylpyrazol aus Beispiel 1 wurden in 20 mL des entsprechenden Lösungsmittels gelöst und unter Eisbad-Kühlung tropfenweise mit einem Äquivalent Säure versetzt. Als Lösungsmittel wurden Aceton, Ethanol und iso-Propanol, als Säurekomponenten ortho-Phosphorsäure (85 %ig), Essigsäure, Schwefelsäure und Oxalsäure verwendet. Bei der Verwendung von Schwefelsäure als Säurekomponente wurde kein Versuch mit Aceton als Lösungsmittel unternommen. Im Falle von ortho-Phosphorsäure und Oxalsäure fielen die Salze aus. Diese wurden nach einstündigem Rühren über eine 50 mL-Glasfilternutsche abfiltriert, dreimal mit jeweils 5 mL eiskaltem Lösungsmittel gewaschen und am Rotationsverdampfer bei 40 °C Badtemperatur bis zur Gewichtskonstanz getrocknet. Die nachfolgende Tabelle enthält die Ausbeuten an Säure-Additionssalz in g und in % bezogen auf eingesetztes rohes 3,4-Dimethylpyrazol (als reines 3,4-Dimethylpyrazol angesehen). Die Tabelle zeigt des Weiteren die Ausbeute bezogen auf eingesetztes 2-Butanon (Gesamtausbeute) und das Verhältnis von 3,4-Dimethylpyrazol zu 3-Ethylpyrazol, die jeweils als Säureadditionssalze vorliegen (Isomerenverhältnis). Die erhaltenen Kristalle enthalten alle noch Spuren der zur Kristallisation verwendeten organischen Lösungsmittel (< 1 %).

| Säure | Lösungsmittel | Ausbeute in g | Ausbeute in % | Gesamtausbeute bez.auf Butanon | Isomeren verhältnis |
|---|---|---|---|---|---|
| ortho-Phosphorsäure | Aceton | 7.57 g | 75.0 % | 55,3 % | 99 : 1 |
| ortho-Phosphorsäure | Ethanol | 7.15 g | 70.8 % | 52,3 % | 99 : 1 |
| ortho-Phosphorsäure | iso-Propanol | 7.71 g | 76.4 % | 56,4 % | 99 : 1 |
| Oxalsäure | Aceton | 7.61 g | 78.6 % | 58,0 % | 95.5 : 4.5 |
| Oxalsäure | Ethanol | 4.41 g | 45.5 % | 33,6 % | 97.5 : 2.5 |
| Oxalsäure | iso-Propanol | 6.56 g | 67.8 % | 50,0 % | 97 : 3 |

In den vereinigten Mutterlaugen, die bei den Kristallisationsversuchen unter Zusatz von ortho-Phosphorsäure anfielen, konnte im 1H-NMR-Spektrum, neben einer größeren Menge nicht weiter charakterisierten Nebenprodukte, 3,4-Dimethylpyrazol und 3-Ethylpyrazol in einem Verhältnis von 33 : 67 nachgewiesen werden. Insbesondere durch Zusatz von etwa einem Äquivalent ortho-Phosphorsäure lässt sich also aus dem Rohprodukt 3,4-Dimethylpyrazol als Phosphorsäure-Additionssalz in guter Ausbeute und in hoher Selektivität von 3-Ethylpyrazol, bzw. dessen Phosphorsäure-Additionssalzes, trennen.

### Beispiel 2

### Synthese von 3-Ethyl-4-methylpyrazol

Die Umsetzung gemäß Beispiel 1 wurde wiederholt, wobei jedoch 2-Butanon durch Pentan-2-on ersetzt wurde. Im erhaltenen Rohprodukt lagen 3-Ethyl-4-methylpyrazol und 3-Propylpyrazol im Verhältnis von 75 : 25 vor.

Die detaillierte Versuchsdurchführung war wie folgt:

**Edukte**

| **Nr.** | **Name** | **Menge** | **Gehalt** | **MG** | **Mol** | **Mol %** |
|---|---|---|---|---|---|---|
| 1 | Natriummethylat (30 %ig in Methanol) | 234 g | 30% | 54,02 | 1,300 | 130 |
| 2 | Pentan-2-on | 86,13 g | | 100,16 | 1,000 | 100 |
| 3 | Methylformiat | 84 g | | 60,05 | 1,400 | 140 |
| 4 | Hydrazin Monohydrat | 50,06 g | | 50,06 | 1,000 | 100 |

In einem 2.L-Dreihalskolben mit KPG-Rührer, Anschütz, Innenthermometer, 250 mL-Tropftrichter und Rückflusskühler mit Hahnolive und Blasenzähler wurden unter einer Stickstoffatmosphäre 180 g 1 vorgelegt und innerhalb einer Stunde ein Gemisch aus 86 g 2 und 66 g 3 über den Tropftrichter so zugetropft, dass die Innentemperatur 30 °C nicht überschritt. Hierbei fiel ein weißer Feststoff aus. Es wurde noch zwei Stunden bei 30 °C nachgerührt, und danach bei dieser Temperatur nochmals 54 g 1 innerhalb 15 Minuten sowie anschließend 18 g 3 innerhalb von zehn Minuten zugetropft. Das Reaktionsgemisch wurde über Nacht bei Raumtemperatur gerührt. Das Reaktionsgemisch wurde in einem Eisbad gekühlt und bei T < 10 °C mit 65 mL 50 %iger Schwefelsäure auf pH = 7 eingestellt, wobei der Feststoff zum Teil in Lösung ging. Danach wurde unter Eisbad-Kühlung bei T < 10 °C 50g 4 zugetropft (stark exotherme Reaktion) und durch gleichzeitige Zugabe von 50 %iger Schwefelsäure der pH-Wert zwischen 7 und 8 gehalten (Verbrauch 20 mL). Nach beendeter Zugabe wurde das Eisbad entfernt und das Reaktionsgemisch über Nacht bei Raumtemperatur gerührt. Das Reaktionsgemisch wurde in einen 2 L-Rundkolben überführt, mit destilliertem Wasser nachgewaschen und mit 25 %iger Natronlauge auf pH = 8 eingestellt. Das Methanol wurde bei 30 °C Badtemperatur am Rotationsverdampfer entfernt. Der Rückstand (ca. 300 mL) wurde mit 250 mL destilliertem Wasser und 500 mL Petrolether 40 / 60 versetzt, in einen 2 L-Scheidetrichter überführt und die organische Phase abgetrennt. Die wässrige Phase wurde noch zweimal mit jeweils 200 mL Petrolether 40 / 60 extrahiert. Die vereinigten organischen Phasen wurden einmal mit 150 mL destilliertem Wasser gewaschen, über Natriumsulfat getrocknet und nach Abfiltrieren des Trockenmittels am Rotationsverdampfer bei 40 °C Badtemperatur eingeengt. Der Rückstand (Rohprodukt) wurde bei 40 °C und 10 mbar bis zur Gewichtskonstanz getrocknet.

Ausbeute: 88,88 g gelbes Öl. Das Rohprodukt bestand laut ¹H-NMR-Spektrum, neben mehreren nicht charakterisierten Produkten, aus einem Gemisch von 3-Ethyl-4-methylpyrazol und 3-Propylpyrazol im Verhältnis 75 zu 25.

### Reinigung als Säure-Additionssalz:

86,82 g (0,7881 mol als reines 3-Ethyl-4-methylpyrazol) Rohprodukt wurden in 870 mL Aceton gelöst. Innerhalb von zehn Minuten wurde unter Wasserbad-Kühlung ein Äquivalent 85 %ige ortho-Phosphorsäure (90,86 g, 0,7881 mol) über einen Tropftrichter zugegeben und die so erhaltene Suspension wurde für eine Stunde gerührt. Der Feststoff wurde über eine G4-Glasfiltemutsche abfiltriert, zweimal mit jeweils 200 mL Aceton gewaschen und bei 40 °C bei 10 mbar getrocknet.

Ausbeute: 83,94 g (40,3 % bezogen auf Pentan-2-on) weißer Feststoff. Laut ¹H-NMR-Spektrum bestand das erhaltene Produkt zu 97 % aus 3-Ethyl-4-methylpyrazolium-dihydrogenphosphat und zu 3 % aus 3-Propylpyrazoliumdihydrogenphosphat. Das Produkt enthielt < 0,5 Gew.-% Aceton.

### Zusätzliche Reinigung:

40,0 g des erhaltenen Salzes wurden für eine Stunde in 400 mL Aceton unter Rückfluss erhitzt. Nach Abkühlen auf Raumtemperatur wurde der Feststoff abfiltriert, einmal mit 100 mL Aceton gewaschen und bei 40 °C am Rotationsverdampfer sowie anschließend im Hochvakuum getrocknet.

Ausbeute: 37,99 g (95,0 % bzw. 38,3 % bezogen auf Pentan-2-on) weißer Feststoff. Das Verhältnis von 3-Ethyl-4-methylpyrazoliumdihydrogenphosphat zu 3-Propylpyrazoliumdihydrogenphosphat konnte laut ¹H-NMR-Spektrum auf 98,9 zu 1,1 verbessert werden. Das Produkt enthielt < 0,5 Gew.-% Aceton.

### Beispiel 3

### Synthese von 3,4-Dimethylpyrazol im größeren Maßstab

**Edukte**

| **Nr.** | **Name** | **Menge** | **Gehalt** | **MG** | **Mol** | **Mol %** |
|---|---|---|---|---|---|---|
| 1 | Natriummethylat in Methanol | 1170 g | 30 %ig | 54,02 | 6,50 | 130 |
| 2 | 2-Butanon | 360 g | | 72,11 | 5,00 | 100 |
| 3 | Methylformiat | 420 g | | 60,05 | 7,00 | 140 |
| 4 | Hydrazin Monohydrat | 250,3 g | | 50,06 | 5,00 | 100 |

### Durchführungsbeschreibung

In einem 6 L-Dreihalskolben mit KPG-Rührer, Anschütz, Innenthermometer, 500 mL-Tropftrichter, Anschütz, 1 L-Tropftrichter und Rückflusskühler mit Hahnolive und Blasenzähler wurden unter einer Stickstoffatmosphäre 900 g 1 vorgelegt und bei 40 bis 45 °C Innentemperatur über eine Stunde ein Gemisch aus 360g 2 und 330 g 3 über den Tropftrichter zugetropft. Hierbei fiel ein weißer Feststoff aus. Es wurde noch zwei Stunden bei 42 °C nachgerührt. Anschließend wurden bei dieser Temperatur weitere 270 g 1 innerhalb von 15 Minuten zugetropft und dann innerhalb von zehn Minuten 90 g 3. Danach wurde über Nacht bei Raumtemperatur gerührt. Die Suspension wurde in einem Eisbad gekühlt und bei T < 10 °C mit 150 mL 50 %iger Schwefelsäure auf pH = 7 eingestellt. Hierbei ging der Feststoff zum Teil in Lösung. Anschließend wurde bei T < 10 °C 250g 4 zugetropft (stark exotherme Reaktion) und durch portionsweise Zugabe von 50 %iger Schwefelsäure der pH zwischen 7 und 8 gehalten (Verbrauch 230 mL). Nach beendeter Zugabe wurde das Eisbad entfernt, und es wurde über Nacht bei Raumtemperatur gerührt. Das Methanol wurde bei 40 °C Badtemperatur am Rotationsverdampfer im Vakuum entfernt. Der Rückstand wurde mit zwei Litern destilliertem Wasser versetzt, in einen 6 L-Scheidetrichter überführt und dreimal mit jeweils drei Litern Petrolether 40 / 60 extrahiert. Die vereinigten organischen Phasen wurden in zwei Portionen aufgeteilt und zweimal mit jeweils einem Liter destilliertem Wasser gewaschen, über Natriumsulfat getrocknet, am Rotationsverdampfer eingeengt und bei 40 °C und 10 mbar bis zur Gewichtskonstanz getrocknet.

Ausbeute: 214,7 g (entspräche 2,2334 mol als reinem 3,4-Dimethylpyrazol) gelbes, hochviskoses Öl. Das Verhältnis von 3,4-Dimethylpyrazol zu 3-Ethylpyrazol betrug laut ¹H-NMR-Spektrum 90,0 zu 10,0.

### Reinigung als Säure-Additionssalz

Das Rohprodukt wurde in 800 mL iso-Propanol gelöst und durch langsame Zugabe von einem Äquivalent 85 %iger ortho-Phosphorsäure (255,44 g 2,2334 mol) als 3,4-Dimethylpyrazoliumdihydrogenphosphat kristallisiert. Das ausgefallene Salz wurde über eine G4-Filternutsche abfiltriert, einmal mit wenig iso-Propanol gewaschen und bei 40 °C und 10 mbar getrocknet.

Ausbeute: 331,6 g (34,1 % bezogen auf 2-Butanon) weißer Feststoff. Das Verhältnis von 3,4-Dimethyl- zu 3-Ethylpyrazoliumdihydrogenphosphat lag laut ¹H-NMR-Spektrum bei 99 zu 1. Das Produkt enthielt weniger als 0,5 Gew.-% iso-Propanol.

## Patentansprüche

1. Verfahren zur Herstellung von Säureadditionssalzen der Pyrazole der allgemeinen Formel (I) 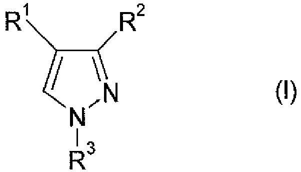 mit der Bedeutung
R¹, R² unabhängig voneinander Wasserstoff, Halogen, Nitro oder gegebenenfalls durch C₁₋₄-Alkyl, Halogen und/oder Nitro substituiertes C₁₋₂₀-Alkyl, C₃₋₈-Cycloalkyl, C₆₋₁₂-Aryl, C₇₋₁₃-Alkylaryl oder C₇₋₁₃-Aralkyl,
R³ Wasserstoff oder gegebenenfalls durch C₁₋₄-Alkyl, Halogen und/oder Nitro substituiertes C₁₋₂₀-Alkyl, C₃₋₈-Cycloalkyl, C₆₋₁₂-Aryl, C₇₋₁₃-Alkylaryl oder C₇₋₁₃-Aralkyl, durch
(1) Umsetzung von Carbonylverbindungen der allgemeinen Formel R¹-CH₂-CO-R² mit den angegebenen Bedeutungen für R¹ und R² in Gegenwart einer starken Base mit Ameisensäureestern der allgemeinen Formel H-COOR⁴, wobei R⁴ C₁₋₈-Alkyl bedeutet, oder mit Kohlenmonoxid,
(2) nachfolgende Umsetzung des gebildeten Zwischenproduktes mit Hydrazinen der allgemeinen Formel R³-NH-NH₂ mit der angegebenen Bedeutung für R³ in Gegenwart anorganischer oder organischer Säuren, und
(3) Lösen der so erhaltenen Pyrazole der allgemeinen Formel (I) in Wasser oder einem organischen Lösungsmittel, nachfolgende Umsetzung mit mindestens äquimolaren Mengen ortho-Phosphorsäure oder Oxalsäure und Abtrennen der erhaltenen Säureadditionssalze durch Kristallisation;
wobei das Zwischenprodukt aus Schritt (1) ohne Abtrennung vom Reaktionsgemisch oder weitere Aufreinigung direkt im Schritt (2) weiter umgesetzt wird.

2. Verfahren nach Anspruch 1, **dadurch gekennzeichnet, dass** aus dem Produkt des Schrittes (2) wasserlösliche Salze der anorganischen oder organischen Säuren abgetrennt werden und das resultierende Produkt im Schritt (3) weiter umgesetzt wird.

3. Verfahren nach Anspruch 1 oder 2, **dadurch gekennzeichnet, dass** die Umsetzung in Schritt (1) bei einer Temperatur im Bereich von -20 bis 70 °C und einem Druck im Bereich von 1 bis 50 bar und die Umsetzung in Schritt (2) bei einer Temperatur im Bereich von 0 bis 90 °C und einem Druck im Bereich von 1 bis 10 bar erfolgen.

4. Verfahren nach einem der Ansprüche 1 bis 3, **dadurch gekennzeichnet, dass** R¹, R² und R³ unabhängig voneinander Wasserstoff oder C₁₋₄-Alkyl bedeuten.

5. Verfahren nach Anspruch 4, **dadurch gekennzeichnet, dass** R¹ und R² unabhängig voneinander Wasserstoff oder Methyl bedeuten und R³ Wasserstoff bedeutet.

6. Verfahren nach einem der Ansprüche 1 bis 5 , **dadurch gekennzeichnet, dass** in Schritt (3) ortho-Phosphorsäure eingesetzt wird.

7. Verfahren nach einem der Ansprüche 1 bis 6, **dadurch gekennzeichnet, dass** das organische Lösungsmittel in Schritt (3) ausgewählt ist aus C₁₋₁₀-Alkanolen, aliphatischen C₃₋₁₀-Ketonen oder Gemischen davon, gegebenenfalls unter Zusatz von Wasser.

8. Verfahren nach Anspruch 7, **dadurch gekennzeichnet, dass** das organische Lösungsmittel ausgewählt ist aus Ethanol, iso-Propanol, Aceton oder Gemischen davon.

## Claims

1. Process for the preparation of acid addition salts of the pyrazoles of the general formula (I): 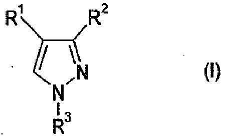 with the meaning
R¹, R², independently of one another, hydrogen, halogen, nitro or C₁₋₂₀-alkyl, C₃₋₈-cycloalkyl, C₆₋₁₂-aryl, C₇₋₁₃-alkylaryl or C₇₋₁₃-aralkyl optionally substituted by C₁₋₄-alkyl, halogen and/or nitro,
R³ hydrogen or C₁₋₂₀-alkyl, C₃₋₈-cycloalkyl, C₆₋₁₂-aryl, C₇₋₁₃-alkylaryl or C₇₋₁₃-aralkyl optionally substituted by C₁₋₄-alkyl, halogen and/or nitro, by
(1) reaction of carbonyl compounds of the general formula R¹-CH₂-CO-R² with the meanings given for R¹ and R² in the presence of a strong base with formate esters of the general formula H-COOR⁴, in which R⁴ means C₁₋₈-alkyl, or with carbon monoxide,
(2) subsequent reaction of the intermediate formed with hydrazines of the general formula R³-NH-NH₂ with the meaning given for R³ in the presence of inorganic or organic acids, and
(3) dissolution of the pyrazoles of the general formula (I) thus obtained in water or an organic solvent, subsequent reaction with at least equimolar amounts of orthophosphoric acid or oxalic acid and separation by crystallization of the acid addition salts obtained;
in which the intermediate from stage (1) is reacted further directly in stage (2) without separation from the reaction mixture or additional purification.

2. Process according to Claim 1, **characterized in that** water-soluble salts of the inorganic or organic acids are separated from the product of stage (2) and the resulting product is reacted further in stage (3).

3. Process according to Claim 1 or 2, **characterized in that** the reaction in stage (1) is carried out at a temperature in the range from -20 to 70°C and a pressure in the range from 1 to 50 bar and the reaction in stage (2) is carried out at a temperature in the range from 0 to 90°C and a pressure in the range from 1 to 10 bar.

4. Process according to any of Claims 1 to 3, **characterized in that** R¹, R² and R³ represent, independently of one another, hydrogen or C₁₋₄-alkyl.

5. Process according to Claim 4, **characterized in that** R¹ and R² represent, independently of one another, hydrogen or methyl and R³ represents hydrogen.

6. Process according to any of Claims 1 to 5, **characterized in that** orthophosphoric acid is used in stage (3).

7. Process according to any of Claims 1 to 6, **characterized in that** the organic solvent in stage (3) is chosen from C₁₋₁₀-alkanols, aliphatic C₃₋₁₀-ketones or mixtures thereof, optionally with addition of water.

8. Process according to Claim 7, **characterized in that** the organic solvent is chosen from ethanol, isopropanol, acetone or mixtures thereof.

## Revendications

1. Procédé pour la préparation de sels d'addition d'acide des pyrazoles de formule générale (I) 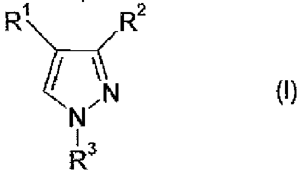 dans laquelle
R¹, R² signifient, indépendamment l'un de l'autre, hydrogène, halogène, nitro ou C₁₋₂₀-alkyle, C₃₋₈-cycloalkyle, C₆₋₁₂-aryle, C₇₋₁₃-alkylaryle ou C₇₋₁₃-aralkyle, le cas échéant substitués par C₁₋₄-alkyle, halogène et/ou nitro,
R³ signifie hydrogène ou C₁₋₂₀-alkyle, C₃₋₈-cycloalkyle, C₆₋₁₂-aryle, C₇₋₁₃-alkylaryle ou C₇₋₁₃-aralkyle, le cas échéant substitués par C₁₋₄-alkyle, halogène et/ou nitro, par
(1) transformation de composés carbonyle de formule générale R₁-CH₂-CO-R², présentant les significations indiquées pour R¹ et R², en présence d'une base forte avec des esters d'acide formique de formule générale H-COOR⁴, R⁴ signifiant C₁₋₈-alkyle, ou avec du monoxyde de carbone,
(2) transformation consécutive du produit intermédiaire formé avec des hydrazines de formule générale R³-NH-NH₂, présentant la signification indiquée pour R³, en présence d'acides inorganiques ou organiques, et
(3) dissolution des pyrazoles ainsi obtenus de formule générale (I) dans de l'eau ou dans un solvant organique, transformation consécutive avec au moins des quantités équimolaires d'acide orthophosphorique ou d'acide oxalique et séparation des sels d'addition d'acide obtenus par cristallisation ;
le produit intermédiaire de l'étape (1) étant transformé davantage, sans séparation du mélange réactionnel ni autre purification, directement dans l'étape (2).

2. Procédé selon la revendication 1, **caractérisé en ce qu'**on sépare du produit de l'étape (2) les sels solubles dans l'eau des acides inorganiques ou organiques et le produit résultant est transformé davantage dans l'étape (3).

3. Procédé selon la revendication 1 ou 2, **caractérisé en ce que** la transformation dans l'étape (1) est réalisée à une température dans la plage de -20 à 70°C et à une pression dans la plage de 1 à 50 bars et la transformation dans l'étape (2) est réalisée à une température dans la plage de 0 à 90°C et à une pression dans la plage de 1 à 10 bars.

4. Procédé selon l'une quelconque des revendications 1 à 3, **caractérisé en ce que** R¹, R² et R³ signifient, indépendamment les uns des autres, hydrogène ou C₁₋₄-alkyle.

5. Procédé selon la revendication 4, **caractérisé en ce que** R¹ et R² signifient, indépendamment l'un de l'autre, hydrogène ou méthyle et R³ signifie hydrogène.

6. Procédé selon l'une quelconque des revendications 1 à 5, **caractérisé en ce qu'**on utilise, dans l'étape (3), de l'acide orthophosphorique.

7. Procédé selon l'une quelconque des revendications 1 à 6, **caractérisé en ce que** le solvant organique dans l'étape (3) est choisi parmi les C₁₋₁₀-alcanols, les C₃₋₁₀-cétones aliphatiques ou leurs mélanges, le cas échéant avec addition d'eau.

8. Procédé selon la revendication 7, **caractérisé en ce que** le solvant organique est choisi parmi l'éthanol, l'iso-propanol, l'acétone ou leurs mélanges.
